# EUROPEAN PATENT APPLICATION

(11) **EP 2 388 021 A1**
(43) Date of publication of application: **23.11.2011**
(21) Application number: 09838422.5
(22) Date of filing: 02.02.2009
(51) Int. Cl.: A61L 27/36

(54) **CONCRETE SCAFFOLD CONTAINING BMP-2 AND MADE OF BONE POWDER AND FIBRIN GLUE**

(30) Priority: 16.01.2009 KR 20090003893
(71) Applicant: Industry-Academic Cooperation Foundation Wonkwang University, Jellabuk-do 570-749 (KR)
(72) Inventor: LEE, Jun, Daejeon-si 305-755 (KR); YOON, Dong-Hyeon, Gumi-si Gyeongsangbuk-do 730-040 (KR)
(74) Representative: Lecca, Patricia S.
(86) International application number: PCT/KR2009/000505
(87) International publication number: WO 2010/082701

(57) **Abstract**

The present invention relates to a bone-regenerating scaffold containing bone morphogenic protein 2 (BMP-2), and more particularly, to a bone-regenerating scaffold which is made of a mixture of fibrin glue and bone powder, the interior of which has a plurality of pores for accommodating bone growth factors, and which has a predetermined concrete shape. The bone-regenerating scaffold of the present invention contains BMP-2 as the above-mentioned bone growth factor.

## Description

### [Technical Field]

The present invention relates to a scaffold for bone regeneration containing bone morphogenic protein 2 (BMP-2), and more particularly, to a scaffold for bone regeneration containing BMP-2 as a bone growth promoting factor, which includes fibrin glue, bone powder mixed with the fibrin glue, and a plurality of pores formed to accommodate the bone growth promoting factor, wherein the scaffold has a predetermined concrete shape, and a method of preparing the scaffold.

### [Background Art]

In recent years, the development of tissue engineering leads to the possibility of developing good graft materials and grafting methods, which can overcome the limitation of autogenous bone. That is, in 1993, Langer and Vacanti reported "injectable tissue engineered bone" (Langer R, Vacanti J P, Tissue engineering. Science 260; 920-926, 1993). Since then, Tayapongsak et al. used an autologous fibrin adhesive for the reconstruction of lower jawbone in 1994 and Marx et al. used platelet-rich plasma to graft bone into patients suffering from lower jawbone defects in 1998. Therefore, these tissue engineering techniques have been recognized as major tools to artificially promote the bone regeneration. In particular, the research of using a bone morphogenic protein, which has been developed since 1965 from the Urist's research, has played the most critical role in the progress of the tissue engineering (Boyne P, et al. Int J. Periodontics Restorative Dent 17:11-25, 1997).

In order to the tissue regeneration, cells that are directly involved in the tissue regeneration, signaling molecules that induce these cells from a living body and allow the cells to differentiate, and a scaffold in which these cells are grown and maintained are required. Therefore, the tissue engineering for bone tissue regeneration aims at preparing a bone scaffold, which has excellent bone-forming capability compared to autogenous bone, by artificially producing such three components and suitably combining these components.

In cytological research, a method of collecting undifferentiated mesenchymal stem cells in a human body to differentiate into osteoblasts and administering or grafting the osteoblasts together with a suitable scaffold, thereby enhancing the bone regeneration capability and improving the success rate of bone grafting has been actively studied (Zvaifler NJ, et al. Arthritis Res 2: 477- 488, 2000; Bianchi G et al. Wound Repair Regen 9: 460-466, 2001; Lennon DP, et al. Exp Cell Res 219: 211-222, 1995; Scott PB, et al. Clin Orthop 355s: s247-s256,1998).

Moreover, extensive research on the bone regeneration effects using various kinds of the growth factors as signaling molecules, which facilitate the induction and differentiation of the osteoblasts, such as a bone morphogenic protein (BMP), a platelet-derived growth factor (PDGF), a vascular endothelial growth factor (VEGF), etc. Among them, BMP-2 has been known to play an important role in the regeneration of bone tissues, and thus much research on the use of the BMP-2 has been in progress (Lieberman JR, et al. J Orthop Res. 16(3):330-339,1998; Yamashita H et al. J Cell Biol. 130(1):217-226,1995; Boyne PJ J Bone Joint Surg Am. 83-A Suppl 1(Pt2):S146-150, 2001; Khan SN et al. Expert Opin Biol Ther. 4(5):741-748. Review, 2004; Friedenstin A et al. Transplantation 12(2):99-108, 1971).

However, there is little research aimed at developing a scaffold that can preserve and maintain signaling molecules containing osteoblasts and various proteins. The reason for this is that there are few scaffolds, each serving as a medium in which living osteoblasts can grow and having the capability to stably maintain the space in which the scaffold is grafted into a bone defect area during healing process. For example, it is known that when BMP-2 is merely injected to a bone defect area, the effects of BMP-2 are maintained only for several hours to several days and the BMP-2 is then degraded. As a result, the effects of the BMP-2 do not last during the recovery of the bone defect area, which takes several weeks to several months.

### [Disclosure]

### [Technical Problem]

The present inventor has made a great effort to develop a scaffold which can overcome the above-described problems, and found that a solid scaffold containing BMP-2, which is obtained by mixing fibrin glue and bone powder and freeze-drying the resulting mixture, can continuously release BMP-2 during bone reconstruction and induce rapid bone regeneration. Accordingly, the present invention has been completed based on these facts.

Therefore, an object of the present invention is to provide a scaffold for bone regeneration containing BMP-2 as a factor for promoting bone growth, the scaffold for bone regeneration including fibrin glue, bone powder mixed with the fibrin glue, and a plurality of pores formed to accommodate a bone growth promoting factor, and having a predetermined concrete shape.

### [Technical Solution]

A "bone morphogenic protein 2 (BMP-2)" is a protein that is known to play an important role in the regeneration of bone tissues. In the present invention, BMP-2 may be derived from a mammal, preferably a human. Preferably, BMP-2 may be derived from the same subject to be treated with BMP-2.

In the present invention, BMP-2 is preferably produced based on a recombinant DNA technology. In one aspect, BMP-2 may be produced by (a) inserting a DNA sequence coding for BMP-2 into a vector including at least one expression control sequence, the vector being operationally connected to the DNA sequence to control the expression of the BMP-2, (b) transforming a host with the resulting recombinant expression vector, (c) culturing the resulting transformant in a suitable culture medium under suitable culture conditions to express the DNA sequence, and (d) isolating the BMP-2 from the culture medium.

The term "vector" refers to a DNA construct containing a DNA sequence operationally connected to a suitable control sequence to express the DNA sequence in a suitable host. The vector may be a plasmid, a phage particle or simply a potential genomic insert.

The "control sequence" means a nucleic acid sequence that is essential or advantageous for the expression of BMP-2. The control sequence includes a promoter, an upstream activating sequence, an enhancer, a polyadenylation sequence, a transcription terminator, etc.

The "host cell" includes known eukaryotic and prokaryotic hosts such as *Escherichia coli* (E. *coli*), *Pseudomonas* sp., *Bacillus* sp., *Streptomyces* sp., fungus, and yeast, insect cells such as *Spodoptera frugiperda,* animal cells such as CHO and mouse cells, tissue-cultured human and plant cells, etc.

The above-described transformation or transfection may be performed according to the methods as described in the basic experimental procedure (Davis et al. Basic Methods in Molecular Biology, 1986). Preferred examples of the method include electroporation, transduction, calcium phosphate transfection, cationic lipid-mediated transfection, etc.

Host cells may be cultured in a suitable culture medium under suitable culture conditions where BMP-2 can be expressed and/or isolated. The cell culturing is performed using a known technique in a suitable nutrient medium containing carbon and nitrogen supply sources and an inorganic salt. A suitable culture medium is commercially available and may be prepared from the components and their composition ratio described in the catalogue of the American Type Culture Collection (ATCC), for example.

BMP-2 may be isolated from a culture using a method known in the art. For example, the BMP-2 may be isolated from a culture by a method including, but is not limited to, centrifugation, filtration, extraction, spray drying, evaporation, or precipitation. Moreover, the BMP-2 may be purified by various methods known in the art such as chromatography or electrophoresis.

The scaffold according to the present invention is **characterized in that** it has a solid and concrete shape. For this purpose, it is preferred that the fibrin glue be mixed with the bone powder and freeze-dried. Moreover, the scaffold according to the present invention may be treated to have a predetermined shape before being freeze-dried, or may be freeze-dried in a predetermined cast. In one aspect, the shape and the cast may correspond to a jawbone or tooth defect area of a patient. More particularly, the cast may be prepared by (a) preparing a 3-dimensional (3D) mold using 3D CT and (b) preparing a cast for preparation of a scaffold suitable for the bone defect area in the 3D mold using a dental resin.

In the present invention, the term "bone powder" refers to a ground bone powder, preferably a ground bone (inorganic) powder, from which osteoblasts are removed. The bone powder may be derived from at least one bone selected from the group consisting of autogenous bone, allogeneic bone, xenogeneic bone, and synthetic bone (for example, hydroxyapatite). In the present invention, the bone powder is commercially available from, for example, Dynagraft (Austem Co. Ltd.), Biocera (Oscotec Inc.), Bio-Oss (Jungsan Biomed Co. Ltd.), ICB (Purgo), MBCP (Purgo), etc.

In the present invention, the term "fibrin glue" refers to a biocompatible and biodegradable product including fibrinogen and thrombin as main components. The fibrin glue has been used in a variety of applications. For example, the fibrin glue has been clinically applied for substitution or reinforcement of sutures by applying fibrinogen, thrombin, calcium chloride, or a fibrinolytic enzyme inhibitor as a tissue adhesive to suture peripheral nerves and fine blood vessels through tissue agglutination of fibrin in Europe. Moreover, in Japan, the fibrin glue has been used as a surgical adhesive for the cerebral nerve surgery including a vascular surgery field, the orthopedic surgery such as bone adhesion, and the arrest of bleeding in patients suffering from lacerated wound, etc. For example, the trade names, such as Greenplast (Green Cross Corp.), Beriplast-P (Aventis) and Tisseel (Baxter), are commercially available.

The fibrin glue according to the present invention preferably includes fibrinogen and thrombin. The fibrinogen may be used in a concentration of 10 to 1000 mg/ml, and preferably 10 to 100 mg/ml. The thrombin may be used in a concentration of 0.1 to 1000 IU/ml, and preferably 1 to 100 IU/ml.

The fibrin glue according to the present invention may further include aprotinin or calcium chloride. Moreover, the fibrin glue according to the present invention may further include a water-soluble binder. The water-soluble binder may be a cell culture medium, distilled water, or blood.

In the present invention, when bone powder and fibrin glue are mixed together, an increase in the amount of the fibrin glue may increase the possibility of causing the toxicity, and therefore it is preferred to suitably adjust the content of the fibrin glue. Moreover, when the size of the scaffold is larger, it is preferred to increase the content of the bone powder to maintain the intensity and shape of the scaffold. In view of these facts, the fibrin glue and the bone powder may be mixed in a volume ratio of 1:1 to 10, preferably 1:1 to 5, and more preferably 1:1 to 3 in the present invention.

The bone growth promoting factor according to the present invention may include a variety of factors for promoting bone growth in addition to the BMP-2. For example, the bone growth promoting factor may include a hormone, a cytokine other than the BMP-2, a stem cell, etc. More particularly, the bone growth promoting factor may be a platelet-derived growth factor (PDGF) or a vascular endothelial growth factor (VEGF).

Since a large number of pores are formed in the mixture of fibrin glue and bone powder while the mixture is subjected to a freeze-drying process, the scaffold according to the present invention may improve absorption and maintenance of the bone growth promoting factor such as BMP-2. The freeze-dried scaffold readily absorbs a medium (or a carrier) containing the bone growth promoting factor and transfers the medium into the pores.

### [Advantageous Effects]

The scaffold for bone regeneration containing BMP-2 according to the present invention can continuously release BMP-2 during bone reconstruction, thereby achieving the more rapid bone regeneration.

### [Description of Drawings]

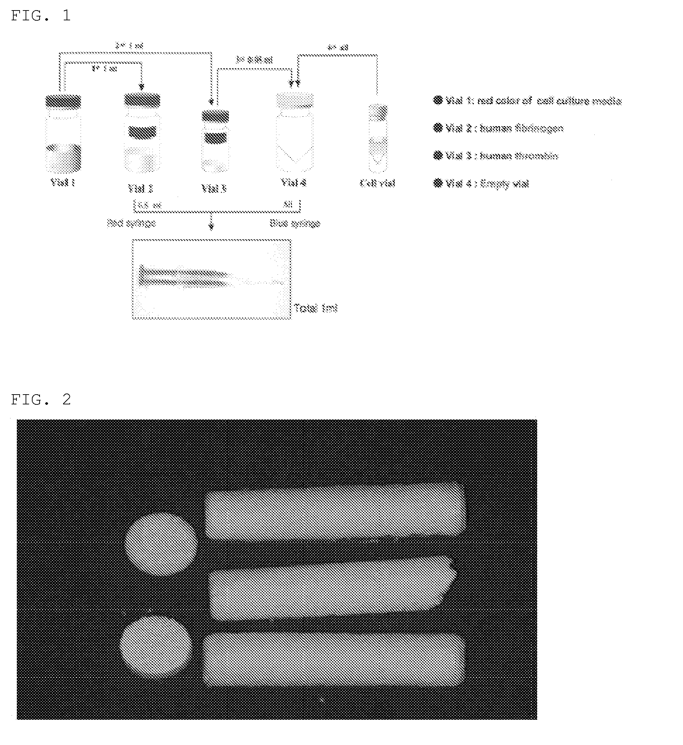

FIG. 1 shows a configuration of a fibrin glue kit used in the present invention.
FIG. 2 shows a freeze-dried Biocera/fibrin block according to the present invention.
FIG. 3 shows a process of preparing a freeze-dried MBCP/fibrin block according to the present invention (A: Mixture of MBCP and fibrin glue, B: Gel state before freeze-drying process, C: Freeze-drying process, D: Solid state after freeze-drying process).
FIG. 4A shows a mixture of MBCP and fibrin glue and FIG. 4B shows a state of an MBCP/fibrin block obtained by freeze-drying the mixture.
FIG. 5 shows the induction of anesthesia of a miniature pig as an experimental animal, the extraction of teeth and the extracted teeth, respectively.
FIG. 6 shows the positions in which a bone graft material according to the present invention is grafted in an alveolus defect area of a miniature pig.
FIG. 7 shows a process of grafting a bone graft material according to the present invention (A: Exposure of a grafted area by flap reflection of a residual alveolar ridge in a lower jaw, B: Formation of only a bone defect area in the form of a saddle, and C: Grafting of a mixture of fibrin glue and MBCP (control) or a fibrin block).
FIGS. 8A to 8D show bone density analysis using a Dicom viewer program (FIG. 8A: Adjustment of lines, FIG. 8B: Adjustment of thickness, FIG. 8C: Measurement of density, and FIG. 8D: Measurement of density divided by 3 fragments (10x10 pixel size, 3 mm thickness)).
FIG. 9 shows the results of 3D computer tomography with respect to the areas in which a bone graft material according to the present invention is grafted.
FIG. 10 shows the results of immunohistochemical staining with respect to the areas in which a bone graft material according to the present invention is grafted.

### [Mode for Invention]

Hereinafter, preferred embodiments of the present invention will be described with reference to the accompanying drawings.

Hereinafter, exemplary embodiments of the present invention will be described in detail below with reference to the accompanying drawings such that those skilled in the art to which the present invention pertains can easily practice the present invention.

### Example 1: Materials and Methods for Experiments

### 1-1. Experimental Animals and Materials

Four 30 kg male miniature pigs (PWG, Korea) were used as experimental animals. The pigs were bred with soft animal feed and water at room temperature in certified breeding facilities for a predetermined period of time.

### 1) Tooth Extraction

Teeth from a premolar tooth to a first molar tooth were extracted from the left and right lower jaws of a miniature pig. After the extraction of the teeth, the wounds were continuously sutured. One week after the suturing process, an injection was performed, and the wounds were healed for one month. During the healing process, additional extraction of impacted teeth was performed during surgery.

### 2) Preparation of MBCP/Fibrin Block

### (1) Preparation of Fibrin Glue

Fibrin glue was prepared using a Greenplast kit® (Greenplst kit, Green Cross Corp., Korea). The 1 ml Greenplast kit includes concentrated human fibrinogen, aprotinin, human serum thrombin, and α-MEM (a medium solution). The aprotinin was mixed with the concentrated human fibrinogen to prepare a fibrinogen solution, and the µ-MEM was mixed with the human serum thrombin to prepare a thrombin solution.

### (2) Preparation of MBCP/Fibrin Complex (hereinafter referred to as "MBCP+fibrin glue")

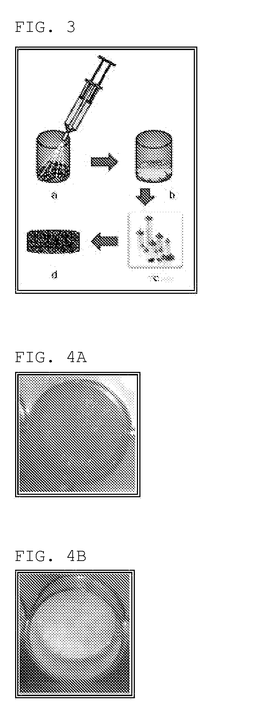

The previously prepared fibrin glue (containing the fibrinogen solution and the thrombin solution) was mixed with MBCP in a volume ratio of 1:1 and the resultant mixture was filled in a previously prepared cast. Then, the mixture was subjected to a freeze-drying process to prepare a MBCP/fibrin block (i.e., a scaffold of the present invention) (FIGS. 3 and 4).

### 1-2. Experimental Methods

### 1) Anesthesia Induction

For bone grafting of a miniature pig, each of an animal anesthetic (Rompun® 3 mg/kg, Bayer Korea Co., Ltd., Korea) and ketamine was intravenously injected to the miniature pig to induce general anesthesia. Oral intubation was performed to induce general anesthesia using N₂O + O₂.

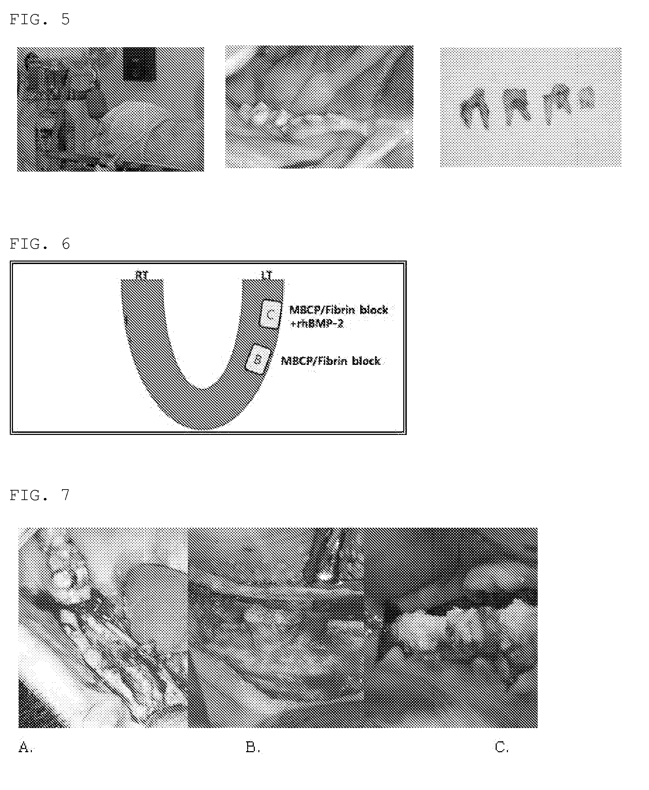

In order to provide a field of vision during the surgery, dogteeth of the upper and lower jaws were tied with a bandage to induce a maximum opening degree. The oral cavity was sterilized with Potadine, and 2% lidocaine (Yuhan Co, Ltd., Korea) containing epinephrine in a volume ratio of 1:100,000 was injected into the residual alveolar bone of the lower jaw to induce local anesthesia and stop bleeding (FIG. 5).

### 2) Alveolar Bone Exposure

The residual alveolar bone of the miniature pig was subjected to horizontal incision and vertical incision, and the periosteum was peeled off to expose the buccal and lingual sides of the lower jaw to the maximum.

### 3) Formation of Control and Experimental Groups

Saddle type 2 wall-bony defects with a depth of 4 to 5 mm, a length of 8 mm, and a base width of 6 to 7 mm were formed using a micro-saw having a radius of 3 mm. Rough regions were polished into a desired shape using a surgical round bur or chisel or a mallet.

First, a MBCP/fibrin block serving as the control was filled in the front of a left-hand bone defect area. Then, recombinant (rh) BMP-2 was soaked in the MBCP/fibrin block and filled in the front of a rear bone defect area (FIG. 6). In this case, the rhBMP-2 was used in a 0.25cc bottle manufactured by COWELLMEDI Co., Ltd.

Releasing incision was performed on the wound to release the tension, and a 4-0 nylon was used to perform a continuous locking suture. Amoxicillin (Tiramox®, SAMJIN Pharmaceutical Co., Ltd., Korea) and diclofenac sodium (Kinpoin®, SAMJIN Pharmaceutical Co., Ltd., Korea) were intramuscularly injected into all experimental animals for three days to prevent infections after surgery, and the experimental animals were fed a soft diet with high-protein milk for one week. Disinfection was performed using chlorhexidine (Hexamedin®, Bukwang Pharmaceutical Co., Ltd., Korea) for one week (FIG. 7).

### 1-3. Visual Inspection

After surgery, disinfection of feeds provided to miniature pigs was continuously performed, and the miniature pigs were maintained under healthy conditions. 2, 4 and 8 weeks after the surgery, the miniature pigs were sacrificed, and the healing level of the bone graft area, the inflammatory condition, and the wound dehiscence were observed with the naked eye before the bone tissue samples were immobilized with formalin.

### 1-4. Computer Tomographic Inspection

Subsequently, each bone graft area was examined using a standard digital radiograph and subjected to dental computer tomography. Afterwards, vertical and horizontal bone cuttings between bone graft areas were performed with respect to the center of the bone graft areas.

### 1) 3D Computer Tomography

The lower jawbone of the miniature pig containing each graft material was imaged using a Cone-beam CT (Alphard vega, Asahi Roentgen Ind. Co., Japan). The imaging was performed at a tube voltage of 80 KVp and a tube current of 8 mA for 15 seconds. Spatial resolutions of all specimens were set to 0.2 mm x 0.2 mm, and approximately 512 images per specimen were obtained. The obtained images were stored in ".dcm" formats, and these files were analyzed using a DICOM Viewer program (OnDemand 3 application, Cybermed, Korea), and all the images were identified and analyzed on a diagnostic monitor (WIDE, Korea).

### 2) Bone Density Analysis

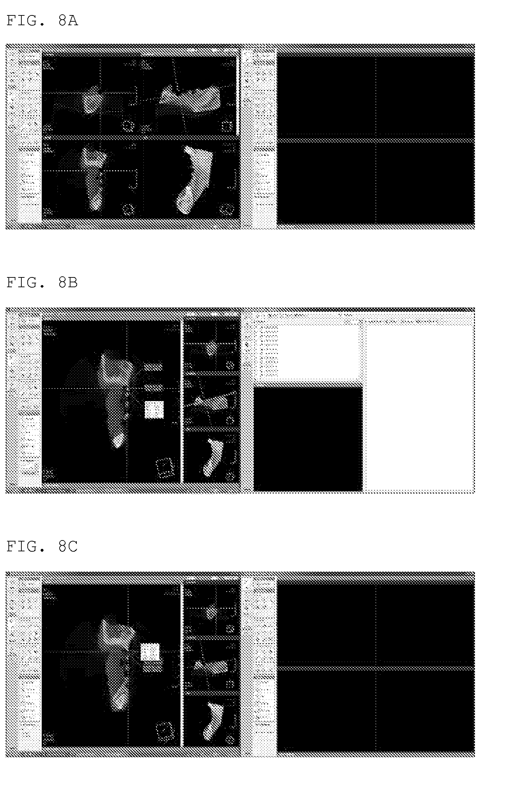

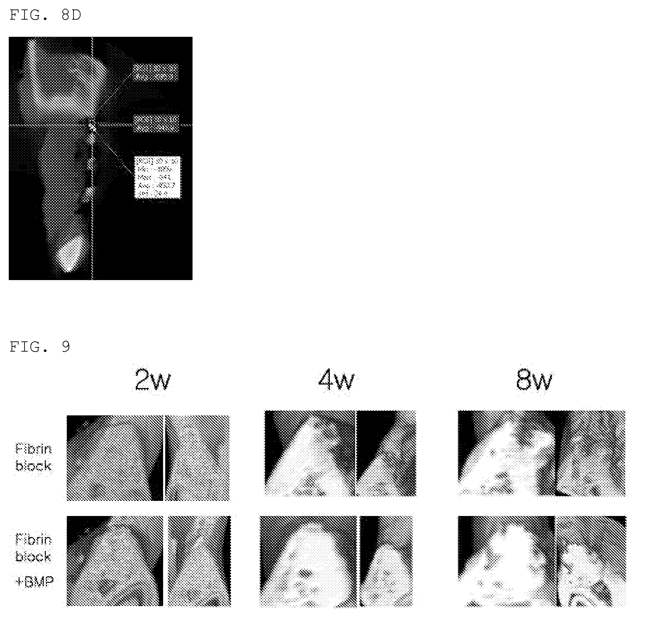

The axes of the coronal plane, the sagittal plane and the transverse plane were adjusted with respect to the center of the bone defect area on the Dicom viewer program. The radiographic opacity of five regions such as center, up, down, left and right regions of the bone defect area on the transverse plane were measured with respect to the region of interest (ROI) of 10 pixel x 10 pixel. Three regions for each bone defect area such as front, middle and rear regions were measured, and the same analysis was performed on each miniature pig for 2, 4 and 8 weeks. Then, the radiographic opacity was statistically analyzed over time. Moreover, a change in radiographic opacity between experimental groups over time was statistically analyzed based on the analysis results. Here, the numerical values on this program were relative values representing the opacities ranging from - 1023 to 3071, and the cortical bone and the cancellous bone of the miniature pig were measured at a radiographic opacity of approximately 500 to 1300 and -100 to 600, respectively (FIG. 8).

### 1-5. Histological Inspection

Miniature pigs were sacrificed 2, 4 and 8 weeks after the surgery to obtain respective tissue samples. Then, the tissue samples were fixed in a 10 % neutral formalin solution for two days, demineralized with 10 % EDTA, and dehydrated and embedded in resin by a typical method. Then, 4 to 6 µm-sized microtomed samples were attached to a poly-L-lysine-coated slide to prepare samples. Tissue sections were prepared so that both the bone graft area and the normal area were included in the tissue sections. In order to observe the shapes of the new bone and fibrous tissue and examine the changes in shapes of the new bone and the fibrous tissue, the samples were stained with Hematoxylin & Eosin and Masson's trichrome stains and examined under a microscope.

### Example 2: Experimental Results

### 2-1. Clinical Features

Each of three miniature pigs in each group was analyzed at the time points of 2, 4 and 8 weeks. It can be seen from the results of clinical evaluation that neither inflammation nor wound dehiscence appeared at the time points of 4 and 8 weeks in the control and the experimental groups, and excellent bone regeneration was observed (Table 1).

**[Table 1]**

| | | | | |
|---|---|---|---|---|
| Inflammation by MBCP/Fibrin Block and MBCP/Fibrin Block+rhBMP-2 | | | | |

| **Groups** | **Bone Graft Materials** | **2 Weeks** | **4 Weeks** | **8 Weeks** |
|---|---|---|---|---|
| Control | MBCP/Fibrin Block | - | - | - |
| Experimental Group | MBCP/Fibrin Block+rhBMP-2 | - | - | - |

| | | | | |
|---|---|---|---|---|
| ( - : negative ± : rare + : mild ++ : moderate +++ : intense ) | | | | |

### 2-2. Radiological Features

### 1) 3D Computer Tomographic Evaluation

At the time point of 4 weeks, the bone defects in the buccal side were somewhat observed in the MBCP/fibrin block of the control, but the alveolar ridge maintained its higher and wider shape. Moreover, the alveolar ridge having a higher and wider shape was observed in the experimental group (MBCP/fibrin block+rhBMP-2), compared to the control, and thus the recovery of the jawbones was very excellent (FIG. 9).

### 2) Bone Density Evaluation

In the radiological features, the bone density was increased in both the control and the experimental group during a normal ossification process. Here, the bone density was significantly increased in the experimental group, compared to the control. A higher bone density was observed especially at the time points of 2 and 8 weeks in the freeze-dried MBCP/fibrin block group, compared to the control. This indicates that the initial graft particles were stabilized by blocking the synthetic bone and fibrin at the time point of 2 weeks, and the bone maturation by premature ossification was observed at the time point of 8 weeks (Table 2).

**[Table 2]**

| Changes in Bone Density by Dental CT (% ± Standard Deviation) | | | | |
|---|---|---|---|---|
| **Groups** | **Bone graft material** | **2 Weeks (*)** | **4 Weeks (*)** | **8 Weeks (*)** |
| Control | MBCP/Fibrin Block(*) | 161.17 (± 5.31) | 190.90 (± 2.00) | 234.07 (± 7.18) |
| Experimental Group | MBCP/Fibrin Block +rhBMP-2 | 186.17 (± 5.24) | 197.70 (± 5.27) | 260.03 (± 0.76) |

| | | | | |
|---|---|---|---|---|
| * p < 0.05 | | | | |

### 2-3. Histological Features

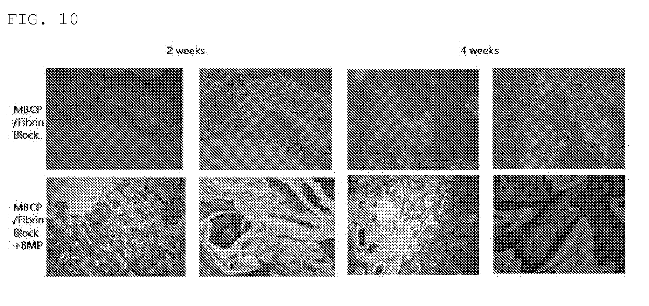

As a result, in the control in which the MBCP/fibrin block was grafted into the bone defect area, the MBCP was scattered in the fibrin structure at the time point of 2 weeks, but new blood vessels appeared while the structure of fibrin was partially destroyed. Moreover, the absorption of the scattered MBCP and the regeneration of new bones around the grafted bone were observed. Furthermore, the activity of osteoblasts was observed more clearly at the time point of 4 weeks, and thus the periphery of the bone defect area was replaced with mature bone. In the experimental group in which the MBCP/fibrin block+rhBMP-2 was grafted into the bone defect area, the absorption of the grafted bone and the regeneration of new bones were active due to the activity of osteoclasts at the time point of 2 weeks, compared to the control, and the entire periphery of the grafted bone was surrounded by the new bone at the time point of 4 weeks (FIG. 10).

In conclusion, saddle-type 2-wall bone defect areas were formed in the lower jawbone of the miniature pig, and the freeze-dried MBCP/fibrin block or the MBCP/fibrin block+BMP-2 was grafted into the bone defect area to compare the osteogenic effects visually, radiologically and histologically at the times points of 2, 4 and 8 weeks after the surgery, and the following comparison results were obtained.
1. In the visual inspection and 3D tomographic inspection, excellent bone regeneration was observed and good bone-grafted shape was maintained in the MBCP/fibrin block+BMP-2-grafted group, compared to the MBCP/fibrin block-grafted group.
2. In the radiological bone density inspection, a higher bone density was measured in the MBCP/fibrin block+BMP-2-grafted group, compared to the MBCP/fibrin block-grafted group, which showed that the bone regeneration was improved.
3. In the histological inspection, the new bone regeneration was highly improved and the bone remodeling progressed more rapidly in the MBCP/fibrin block+BMP-2-grafted group, compared to the MBCP/fibrin block-grafted group.

As described above, it can be seen that the good bone-grafted shape was maintained and the new bone regeneration was highly improved when the freeze-dried MBCP/fibrin block was grafted into the bone defect area. Here, when the freeze-dried MBCP/fibrin block was administered together with BMP-2, it was considered that the bone regeneration was expected to progress more rapidly.

### [Industrial Applicability]

As described above, the scaffold for bone regeneration containing BMP-2 according to the present invention can continuously release BMP-2 during bone regeneration and thus can be used for the rapid bone regeneration.

## Claims

1. A scaffold for bone regeneration containing a bone morphogenic protein 2 (BMP-2) as a bone growth promoting factor, the scaffold comprising:
fibrin glue;
bone powder mixed with the fibrin glue; and
a plurality of pores formed to accommodate the bone growth promoting factor,
wherein the scaffold has a predetermined concrete shape.

2. The scaffold according to claim 1, wherein the BMP-2 is produced by (a) inserting a DNA sequence coding for the BMP-2 into a vector including at least one expression control sequence, the vector being operationally connected to the DNA sequence to control the expression of the BMP-2, (b) transforming a host with the resulting recombinant expression vector, (c) culturing the resulting transformant in a suitable culture medium under suitable culture conditions to express the DNA sequence, and (d) isolating the BMP-2 from the culture medium.

3. The scaffold according to claim 1, wherein the scaffold is treated to have a predetermined shape before being freeze-dried.

4. The scaffold according to claim 1, wherein the scaffold is freeze-dried in a predetermined cast.

5. The scaffold according to claim 4, wherein the cast is prepared by (a) preparing a 3-dimensional (3D) skull mold using 3D computed tomography (CT) and (b) preparing a cast for preparation of the scaffold suitable for a bone defect area using a dental resin in the 3D skull mold.

6. The scaffold according to claim 1, wherein the bone powder is a ground bone powder from which osteoblasts are removed.

7. The scaffold according to claim 6, wherein the bone powder is derived from at least one bone selected from the group consisting of autogenous bone, allogeneic bone, xenogeneic bone, and synthetic bone.

8. The scaffold according to claim 1, wherein the fibrin glue comprises fibrinogen and thrombin.

9. The scaffold according to claim 8, wherein the fibrinogen is present in a concentration of 10 to 1000 mg/ml.

10. The scaffold according to claim 8, wherein the thrombin is present in a concentration of 0.1 to 1000 IU/ml.

11. The scaffold according to claim 1, wherein the fibrin glue further comprises aprotinin or calcium chloride.

12. The scaffold according to claim 1, wherein the fibrin glue further comprises a water-soluble binder.

13. The scaffold according to claim 12, wherein the water-soluble binder is a cell culture medium, distilled water, or blood.

14. The scaffold according to claim 1, wherein the bone powder and the fibrin glue are mixed in a volume ratio of 1 to 10:1.

15. The scaffold according to claim 1, wherein the bone growth promoting factor is a hormone, a cytokine, or a stem cell.
